# FASCICULE DE BREVET EUROPEEN

(11) **EP 0 537 838 B1**
(45) Date de publication et mention de la délivrance du brevet: **28.12.1994**
(21) Numéro de dépôt: 92203075.4
(22) Date de dépôt: 06.10.1992
(51) Int. Cl.: B01J 37/04, B01J 23/96, B01J 23/44, C07C 51/377

(54) **Catalyseurs de déshalogénation d'acides carboxyliques alphahalogènes**
Katalysatoren zur Enthalogenierung von Alpha-halogenierten Karbonsäuren
Catalysts for the dehalogenation of alpha-halogenated carboxylic acids

(30) Priorité: 18.10.1991 FR 9112915
(43) Date de publication de la demande: 21.04.1993
(73) Titulaire: ELF ATOCHEM S.A., 92800 Puteaux (FR)
(72) Inventeur: Correia, Yves, F-04160 Château Arnoux (FR); Nowocien, Joseph, F-04160 Château Arnoux (FR); Jourdain, Dominique, F-04600 Saint-Auban (FR); Salerno, Alexandre, F-04160 Château Arnoux (FR)

(56) Documents cités:
- FR-A- 2 046 424
- FR-A- 2 196 196
- FR-A- 2 645 531
- US-A- 4 422 954
- US-A- 4 888 316

## Description

La présente invention concerne un catalyseur de deshalogénation d'acides carboxyliques alphahalogénés. L'invention concerne particulièrement un catalyseur pour éliminer l'acide dichloracétique (ADCA) contenu dans l'acide monochloracétique (AMCA). La synthèse de l'acide monochloracétique à l'échelle industrielle se fait par la chloration de l'acide acétique , mais il se forme inévitablement de l'acide dichloracétique et parfois un peu d'acide trichloracétique. On obtient donc de l'acide monochloracétique brut qui est un mélange constitué d'acide monochloracétique, d'acide dichloracétique, de traces de trichloractétique et d'acide acétique qui n'a pas réagi. Compte tenu de la proximité des points d'ébullition de l'AMCA (189 °C) et de l'ADCA (194 °C), il est pratiquement impossible de les séparer par distillation. Par contre, il est très simple d'hydrogéner ce mélange pour convertir l'ADCA en AMCA selon la réaction :

CHCl₂COOH + H₂ ---> CH₂ClCOOH + HCl

Cette hydrogénation n'est pas totalement sélective et on observe aussi une rétrogradation d'AMCA en acide acétique :

CH₂ClCOOH + H₂ ---> CH₃COOH + HCl

Cette réaction se fait avec un catalyseur et sous-produit de l'acétaldéhyde qui a l'inconvénient de générer des produits de condensation.

L'hydrogénation de l'acide monochloracétique brut produit aussi des lourds tels que du monochloracétate de l'acide glycolique (MAAG).

L'hydrogénation de l'acide monochloracétique brut se fait en présence de palladium déposé sur du charbon, de l'alumine ou de la silice. Ce procédé est décrit par exemple dans les brevets US 2863917 et GB 1188745. On a aussi décrit dans le brevet FR 2647032 le dopage de ces catalyseurs au palladium par du soufre ou des composés soufrés. Le brevet FR 2046424 décrit un procédé de régénération de ces catalyseurs au palladium. En effet, ces catalyseurs au cours de leur fonctionnement perdent de l'activité, de la sélectivité et génèrent plus d'aldéhydes et de produits lourds. Selon FR 2046424 on oxyde par du chlore gazeux entre 100 et 150 °C le catalyseur usagé pour convertir le palladium en son chlorure puis on réduit par l'hydrogène ou d'autres agents réducteurs pour retrouver le palladium métallique.

La demanderesse a trouvé un procédé très simple pour regénérer ces catalyseurs c'est-à-dire à partir d'un catalyseur usagé obtenir un catalyseur ayant les propriétés d'un catalyseur neuf.

La présente invention est donc un mélange de :
a) Un métal précieux du groupe 8 déposé sur un support inerte et ayant été utilisé pour deshalogéner des acides carboxyliques alphahalogènes en présence d'hydrogène.
b) Un métal précieux du groupe 8 déposé sur un support inerte
et tel que la granulométrie de b soit très inférieure à celle de a pour former un mélange homogène sous l'effet des courants d'acide carboxylique alphahalogèné et d'hydrogène.

Brièvement résumée l'invention consiste à rajouter à un catalyseur usagé de déshalogénation d'acide carboxylique alphahalogéné des fines de catalyseur neuf, les fines se mélangent avec le catalyseur usagé sous l'effet du courant d'acide carboxylique et du courant d'hydrogène formant ainsi un nouveau catalyseur ayant les propriétés d'un catalyseur neuf. Un autre avantage est qu'on économise l'arrêt de la production; la vidange du réacteur et le remplacement de la charge de catalyseur.

Le produit décrit en a est un catalyseur. Les métaux précieux du groupe 8 de la classification périodique des éléments sont le ruthenium, le rhodium, le palladium, l'osmium, l'iridium et le platine. Ce métal est déposé sur un support tel que du charbon, de la silice, du carbure de silicium, d'aluminium ou du carbure de bore. La quantité de métal précieux peut être comprise entre 0,1 et 10 % de l'ensemble métal précieux plus support.

Le métal précieux est avantageusement déposé sur un charbon de grande surface à raison de 0,1 à 5 % en poids du catalyseur, c'est-à-dire du charbon plus du métal et il est réparti en surface du charbon. On entend par charbon de grande surface un charbon d'environ 600 m²/g et pouvant aller jusqu'à 1300 m²/g. Ce charbon se présente sous forme de petits cylindres extrudés ou d'une poudre. De préférence le métal précieux est le palladium.

A titre d'exemple d'acides carboxyliques alphahalogénés on peut citer les acides de formule : 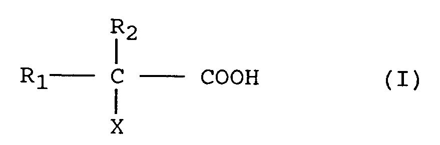
dans laquelle X est le chlore, R₁ et R₂ sont identiques ou différents et représentent X, H, un radical alkyle linéaire ou ramifié ayant de 1 à 12 carbones ou un radical cycloalkyle ayant de 3 à 12 carbones. L'invention s'applique aussi aux esters des acides de formule (I). Ce sont de préférence les esters aliphatiques ayant de 1 à 10 carbones et de préférence 1 à 5 atomes de carbone.

Le produit de a est un catalyseur ayant été utilisé pour deshalogéner des acides carboxyliques alphahalogénés c'est-à-dire qu'il a perdu une partie de ses capacités au fur et à mesure du temps d'utilisation. Cette usure se traduit le plus souvent par une baisse d'activité, il faut donc augmenter la température pour obtenir la même production à masse de catalyseur égale. On observe aussi une augmentation des sous produits. On observe aussi une perte de métal précieux. L'invention concerne en a des catalyseurs ayant été utilisés et que l'on doit changer soit parce qu'ils ne sont plus assez actifs, soit parce qu'ils font trop de sous produits.

Le produit b est un produit comme a et de granulométrie inférieure à celle de a pour pouvoir se mélanger facilement avec a sous l'effet des courants d'acide carboxyliques alphahalogénés et de l'hydrogène et former un ensemble homogène. Il est préférable que b soit actif c'est par exemple des fines de catalyseur neuf ou du catalyseur neuf de faible granulométrie.

La demanderesse a découvert que le mélange de a et b bien que contenant moins de métal précieux que a à l'état neuf avait les mêmes propriétés que a à l'état neuf.

La quantité de b à ajouter à a dépend des performances recherchées. Par exemple, si on veut obtenir un catalyseur ayant les mêmes propriétés et la même production que a à l'état neuf et que a est usagé de telle sorte qu'il a perdu 40 % du métal précieux initial, il suffit d'ajouter une quantité de b pouvant aller de 3 à 10 % de la masse initiale de a et ayant la même teneur initiale en métal précieux. Cette quantité de b peut varier puisqu'on peut utiliser un catalyseur b plus riche ou moins riche en métal précieux que a à l'état neuf. Si on veut obtenir un catalyseur plus actif ou moins actif que a à l'état neuf il suffit d'ajouter plus ou moins de b ou du catalyseur b plus ou moins riche en métal précieux. On ne sortirait pas du cadre de l'invention si le mélange a et b de l'invention contenait plus de métal précieux que a à l'état neuf.

En effet, si a à l'état neuf pesait 10.000 g et contenait 1% de métal précieux c'est-à-dire 100 g, la perte étant par exemple de 40 % il reste 60 g de métal on ajoute 5000 g de b à 1 % soit 50 g de métal précieux. Le mélange a + b contient donc 110 g de métal précieux. On voit donc qu'en rajoutant 5000 g de catalyseur neuf à 10.000 g initiaux de catalyseur qui a fonctionné et est usagé on obtient une nouvelle charge de catalyseur à l'aide d'une quantité de cata égale à la moitié de la charge initiale au lieu de changer toute la charge.

L'invention est particulièrement utile pour purifier des acides carboxyliques monoalphahalogénés R₁CHXCOOH impurs, R₁ ayant la signification précédente. Ces acides sont préparés par halogénation de l'acide correspondant R₁CH₂COOH, on obtient un mélange de R₁CHXCOOH, de R₁CX₂COOH, d'acide R₁CH₂COOH qui n'a pas été transformé et parfois des traces de CX₃ COOH dans le cas particulier de l'acide CH₃COOH.

On pourrait d'abord séparer R₁CH₂COOH de ce mélange mais il est plus simple d'hydrogéner d'abord

R₁CX₂COOH + H₂ ---> R₁CHXCOOH + HX

et séparer ensuite, puisque inévitablement une partie de R₁CHXCOOH est rétrogradée en acide selon :

R₁CHXCOOH + H₂ ---> R₁CH₂COOH + HX

Il suffit ensuite de distiller le mélange de R₁CHXCOOH, de R₁CH₂COOH et d'HX pour obtenir R₁CHXCOOH relativement pur.

L'invention s'applique en particulier à la purification de l'acide monochloracétique.

La rétrogradation est le rapport entre le nombre d'ions X dans l'acide purifié, c'est-à-dire ceux provenant de HX au nombre théorique de X à enlever de R₁CX₂COOH, (et éventuellement CX₃COOH) pour le convertir en R₁CHXCOOH. Sauf exception de CX₃COOH, la rétrogradation minimum est de 1. Cette rétrogradation est le plus souvent entre 1,4 et 3,4.

Cette rétrogradation élevée montre que la réaction n'est pas sélective, on obtient des aldéhydes et des produits lourds.

La demanderesse a constaté qu'en ajoutant des fines de catalyseurs neuf à une charge usagée, c'est-à-dire en formant une nouvelle charge catalytique constituée du mélange a et b selon l'invention, on obtient les propriétés d'une charge de catalyseur neuf.

La présente invention concerne aussi un procédé pour purifier des acides carboxyliques monoalphahalogénés de formule R₁CHXCOOH, R1 ayant la signification précédente caractérisée en ce qu'on utilise une charge catalytique contenant a et b selon l'invention.

### EXEMPLE 1

1/ On charge une colonne A en verre, double enveloppe de 26 mm de diamètre intérieur, avec 130 g d'un charbon extrudé de diamètre 2 mm et longueur 4 mm ayant une surface spécifique > 700 m²/g et contenant 0,8 % de palladium déposé à la surface (> 100 m²/g de palladium).
   Cette colonne est ensuite alimentée à co-courant par une solution d'acide brut (en % poids) : acide monochloracétique environ 80 %, acide dichloracétique environ 4 %_{,} acide acétique environ 16 % et un flux d'hydrogène de 4 normaux litres heures.
   La colonne est portée en température, 125°C, et après une centaine d'heures de marche on obtient les résultats suivants (voir tableau **catalyseur neuf**)
2/ Après plusieurs milliers d'heures de marche le catalyseur devient usagé (voir tableau **catalyseur usagé**) et c'est donc un catalyseur tel a.
   Le catalyseur neuf contenait 0,8 % de palladium soit 1040 mg. Le catalyseur usagé pèse 170 g et contient 0,39 % de palladium soit 660 mg.
   Dans le tableau "ADCA" désigne l'acide dichloracétique, "MAAG" désigne le monochloracétate de l'acide glycolique, "-CHO" désigne les aldéhydes (exprimé en acétaldéhyde) en mg par kg d'acide brut à purifier, 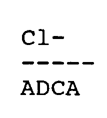 représente la rétrogradation.
   La vitesse spaciale de liquide est la vitesse de l'acide brut par heure et m³ de lit catalytique.
3/ On ajoute ensuite 13 g de fines de catalyseur neuf sous forme d'une poudre de 70 microns constituée de charbon contenant 1 % de palladium, c'est du produit b.

On constate que 3,2 g ressortent du lit catalytique et 9,8 g restent dans le lit catalytique. Cet ajout s'effectue sans arrêter la réaction.

On remet le comptage des heures de marche à zéro et après 282 heures on mesure les résultats (voir tableau catalyseur usagé rénové par ajout de catalyseur en poudre).

On a mesuré les performances jusqu'à 1200 heures, on a donc ajouté au lit catalytique 98 mg. Le mélange selon l'invention contient 660 mg + 98 mg = 758 mg.

On constate alors que l'on peut baisser la température du lit pour avoir des performances sur la conversion de l'acide dichloracitique identiques à celles observées précédemment, les teneurs en sous produits acétaldéhyde et monochloracétate d'acide glycolique (MAAG) sont fortement abaissées par rapport à celles observées avec le catalyseur usagé non réactivé et cette activité se maintient dans le temps.

**TABLEAU**

| | **Heures de marche** | **temp ° C** | **vitesse spaciale liquide kg/h/m³** | **ADCA %** | | **MAAG %** | **CHO mg/kg** | **Cl-** **ADCA** |
|---|---|---|---|---|---|---|---|---|
| | | | | **entrée/sortie** | | | | |
| cata usagé | | 155 | 249 | 3,35 | 0,17 | 1,45 | 825 | 2,67 |
| | | 160 | 261 | 3,35 | 0,14 | 1,40 | 985 | 3,13 |
| cata usagé rénové par ajoût de de cata en poudre | 282 | 140 | 250 | 3,22 | 0,14 | 0,40 | 402,5 | 1,71 |
| | 434 | 140 | 234 | 2,56 | 0,10 | 0,60 | 340 | 2,10 |
| | 651 | 140 | 255 | 2,97 | 0,16 | 0,70 | 243 | 1,78 |
| | 742 | 140 | 242 | 2,95 | 0,14 | 0,80 | 254 | 1,89 |
| | 1200 | 140 | 238 | 3,02 | 0,15 | 0,70 | 290 | 1,84 |
| cata neuf | 200 | 135 | 241 | 3,29 | 0,17 | 0,33 | 354 | 2,96 |
| | 250 | 135 | 272 | 3,09 | 0,13 | 0,40 | 308 | 2,98 |

## Revendications

1. Catalyseur de déshalogénation d'acides carboxyliques alphahalogénés constitué par un mélange de ;
a) Un catalyseur usage constitué d'un metal précieux du groupe 8 déposé sur un support inerte et ayant été utilisé pour déshalogéner des acides carboxyliques alphahalogénés en présence d'hydrogène,
b) Des fines de catalyseur neuf constitué d'un métal précieux du groupe 8 déposé sur un support inerte et tel que la granulométrie de b soit très inférieure à celle de a pour pouvoir former un mélange homogène sous l'effet des courants d'acide carboxylique alphahalogène et d'hydrogène.

2. Catalyseur selon la revendication 1 caractérisé en ce que le métal précieux de a et b est le palladium.

3. Catalyseur selon la revendication 1 ou 2 caractérisé en ce que les acides alphahalogénés sont de formule : 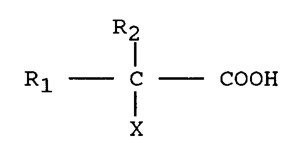 dans laquelle X est le chlore ou le brome, R₁ et R₂ sont identiques ou différents et représentent X, H, un radical alkyle linéaire ou ramifié ayant de 3 à 12 carbones.

4. Procédé de purification d'acides carboxyliques monoalphahalogénés de formule R₁CHXCOOH, R₁ ayant la signification précédente, caractérisé en ce qu'on utilise une charge catalytique selon la revendication 1 ou 2.

5. Procédé selon la revendication 4 caractérisé en ce que l'acide purifié est de l'acide monochloracétique brut.

## Claims

1. Catalyst for the dehalogenation of alpha-halogenated carboxylic acids consisting of a mixture of:
a) a spent catalyst consisting of a group VIII precious metal deposited on an inert support, which has been used for dehalogenating alpha-halogenated carboxylic acids in the presence of hydrogen;
b) fresh catalyst fines consisting of a group VIII precious metal deposited on an inert support, such that the particle size of b is much less than that of a in order to be able to form a homogeneous mixture under the effect of streams of alpha-halogenated carboxylic acid and of hydrogen.

2. Catalyst according to Claim 1, characterized in that the precious metal of a and b is palladium.

3. Catalyst according to Claim 1 or 2, characterized in that the alpha-halogenated acids are of formula: 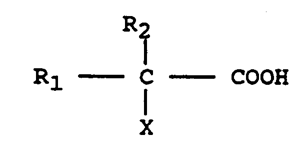 in which X is chlorine or bromine, R₁ and R₂ are identical or different and represent X, H or a linear or branched alkyl radical having from 3 to 12 carbons.

4. Process for the purification of mono-alpha-halogenated carboxylic acids of formula R₁CHXCOOH, R₁ having the above meaning, characterized in that a catalytic charge according to Claim 1 or 2 is used.

5. Process according to Claim 4, characterized in that the acid purified is crude monochloroacetic acid.

## Patentansprüche

1. Katalysator zur Dehalogenierung α-halogenierter Carbonsäuren, gebildet aus einer Mischung von:
a) einem gebrauchten Katalysator, bestehend aus einem Edelmetall der Gruppe 8, das auf einem inerten Träger aufgebracht ist, und zur Dehalogenierung α-halogenierter Carbonsäuren in Gegenwart von Wasserstoff verwendet wurde,
b) Feinteilchen eines neuen Katalysators, der aus einen, Edelmetall der Gruppe 8 besteht, das auf einem inerten Träger aufgebracht ist, wobei die Granulometrie von **b** viel Kleiner als die von **a** ist, um unter dem Einfluß von strömendem Wasserstoff und α-halogenierter Carbonsäure eine homogene Mischung zu bilden.

2. Katalysator nach Anspruch 1, dadurch gekennzeichnet, daß das Edelmetall von **a** und **b** Palladium ist.

3. Katalysator nach Anspruch 1 oder 2, dadurch gekennzeichnet, daß die α-halogenierten Säuren der Formel: 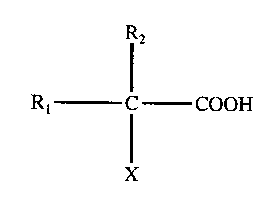 entsprechen, in der X ein Chlor- oder Bromatom ist, R₁ und R₂ identisch oder verschieden sind und X, H, einen linearen oder verzweigten Alkylrest mit 3 bis 5 Kohlenstoffatomen darstellen.

4. Verfahren zur Reinigung mono-α-halogenierter Carbonsäuren der Formel R₁CHXCOOH, wobei R₁ die gleiche Bedeutung wie oben besitzt, dadurch gekennzeichnet, daß man einen Katalysator nach Anspruch 1 oder 2 verwendet.

5. Verfahren nach Anspruch 4, dadurch gekennzeichnet, daß die gereinigte Säure technische Monochloressigsäure ist.
